Europäisches Patentamt

European Patent Office

Office européen des brevets

(19)

(11) **EP 1 188 736 A1**

(12) **EUROPÄISCHE PATENTANMELDUNG**

(43) Veröffentlichungstag:
**20.03.2002 Patentblatt 2002/12**

(51) Int Cl.7: **C07C 29/70**

(21) Anmeldenummer: **01119327.3**

(22) Anmeldetag: **10.08.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **14.09.2000 DE 10045356**

(71) Anmelder: **Degussa AG**
**40474 Düsseldorf (DE)**

(72) Erfinder:
- **Standke, Burkhard, Dr.**
  **79540 Lörrach (DE)**
- **Rauleder, Hartwig, Dr.**
  **79618 Rheinfelden (DE)**
- **Horn, Michael, Dr.**
  **79618 Rheinfelden (DE)**

(54) **Verfahren zur Herstellung von Magnesiummethanolat**

(57) Die vorliegende Erfindung betrifft ein Verfahren zur Herstellung von Magnesiummethanolat durch Umsetzen von Magnesium mit Methanol, das dadurch gekennzeichnet ist, dass man Magnesium in Stückform einsetzt. Ferner betrifft die Erfindung die Verwendung von erfindungsgemäß hergestelltem Magnesiummethanolat als Einsatzstoff für chemische oder pharmazeutische Prozesse oder für die Herstellung von Katalysatoren oder als wasserfreies Neutralisationsmittel.

Abbildung 1

Printed by Jouve, 75001 PARIS (FR)

## Beschreibung

Die vorliegende Erfindung betrifft ein Verfahren zur Herstellung von Magnesiummethanolat sowie dessen Verwendung.

Magnesiumalkoxide kann man bei der Herstellung von Ether verwenden. Hierbei wird ein Magnesiumalkoxid mit einem Alkylhalogenid umgesetzt. Insbesondere werden Magnesiumalkoxide bei solchen Ethersynthesen eingesetzt, bei denen ansonsten unter Verwendung anderer Alkoxide, z. B. Alkalialkoxide, aus den Alkylhalogeniden Chlorwasserstoff abgespalten wird, und so Olefine als Nebenprodukte gebildet werden. Auch kann man ein Dialkylsulfat mit einem Magnesiumalkoholat zu einem Ether umsetzen.

Es ist bekannt, Magnesiumalkoholate aus einem wasserfreien Alkohol und Magnesium in Gegenwart von Quecksilber(ll)-chlorid oder Jod als Katalysator herzustellen [Chem. Abstr. **66,** 37426g (1967)]. Magnesium wird hierbei meist in fein verteilter Form - Pulver oder Späne oder Perlen - eingesetzt. Die Reaktion verläuft jedoch mit manchen Alkoholen, z. B. Methanol, nach verzögertem Reaktionsstart sehr heftig bis unkontrolliert. Dies stellt für die Herstellung von Magnesiummethanolat aus Methanol und Magnesium, insbesondere im technischen Maßstab, ein Problem dar.

Es bestand daher die Aufgabe, ein Verfahren bereit zu stellen, bei dem das Magnesium mit Methanol kontrolliert abreagiert. Ein besonderes Anliegen der vorliegenden Erfindung war es, Magnesiummethanolat in methanolischer Lösung oder in fester Form mit hoher Qualität und guter Ausbeute herzustellen.

Die gestellte Aufgabe wird erfindungsgemäß entsprechend den Angaben der Patentansprüche gelöst.

So wurde ein Verfahren zur Herstellung von Magnesiummethanolat gefunden, bei dem überraschenderweise in einer Reaktionsapparatur Magnesium in Stückform, beispielsweise Barrenform, mit überschüssigem Methanol zu Magnesiummethanolat in einem kontrollierten Prozess in besonders einfacher und wirtschaftlicher Weise umgesetzt wurde, wobei das eingesetzte Magnesium praktisch vollständig abreagierte.

Gegenstand der vorliegenden Erfindung ist daher ein Verfahren zur Herstellung von Magnesiummethanolat durch Umsetzen von Magnesium mit Methanol, das dadurch gekennzeichnet ist, dass man Magnesium in Stückform einsetzt.

Geeigneterweise besitzen die beim erfindungsgemäßen Verfahren eingesetzten Magnesiumstücke im Mittel eine geometrische, d. h. äußere Oberfläche von mindestens $\geq 10\ cm^2$, geeigneterweise von 20 bis 20.000 $cm^2$, vorzugsweise von 40 bis 1.000 $cm^2$, besonders bevorzugt von 50 bis 500 $cm^2$, ganz besonders bevorzugt von 70 bis 300 $cm^2$.

Vorzugsweise besitzt das beim erfindungsgemäßen Verfahren eingesetzte Magnesium Blockform, z. B. Barren, Massel, Kugeln oder Perlen, oder Säulenform, wie z. B. Magnesiumstäbe und Stangen, oder Plattenform, wobei die Mindeststärke bzw. der Mindestdurchmesser der Magnesiumstücke vorzugsweise $\geq 1{,}0\ cm$ beträgt.

In der Regel ist man bestrebt möglichst reines Magnesium einzusetzen. Geeigneterweise besitzt das beim erfindungsgemäßen Verfahren eingesetzte Magnesium einen Magnesiumgehalt von > 99 Gew.-%.

Beim vorliegenden Verfahren eingesetzte Stücke an metallischem Magnesium können vor der Umsetzung zusätzlich von anhaftenden Verunreinigungen befreit werden, beispielsweise das Entfernen einer Oxid-, Hydroxidbzw. Carbonatschicht durch Ätzen oder Polieren oder Schleifen oder Sägen, um nur einige Möglichkeiten zu nennen.

In der Regel setzt man beim erfindungsgemäßen Verfahren das Methanol im Überschuss ein, wobei das Methanol gleichzeitig die Funktion einer Eduktkomponente des Reaktionsmediums sowie eines Lösemittels übernimmt. Vorzugsweise setzt man getrocknetes Methanol ein.

Vorzugsweise führt man beim erfindungsgemäßen Verfahren die Umsetzung der Magnesiumstücke mit Methanol bei einer Temperatur im Bereich von 0 bis 200 °C, besonders vorzugsweise bei 15 bis 70 °C, ganz besonders vorzugsweise bei 50 bis 65 °C, durch. Geeigneterweise führt man die erfindungsgemäße Umsetzung bei einem Druck im Bereich von 0,1 bis 100 bar abs. durch. Bevorzugt führt man die Umsetzung bei 1 bis 20 bar abs., ganz besonders bevorzugt bei 1 bis 5 bar abs., durch.

Die Umsetzung erfolgt beim erfindungsgemäßen Verfahren in der Regel quantitativ, wobei eine methanolische Lösung von Magnesiummethanolat hoher Reinheit anfällt. Sollte beim vorliegenden Verfahren nach einer vorgegebenen, d. h. begrenzten Reaktionsdauer Reste an metallischem Magnesium vorliegen, können diese, beispielsweise durch Filtration, abgetrennt werden.

Vorzugsweise wird das erfindungsgemäße Verfahren so betrieben, dass man als Edukte flüssiges Methanol und festes Magnesium in einem solchen Mengenverhältnis einsetzt, dass daraus eine methanolische Lösung mit einem Gehalt von 0,1 bis 30 Gew.-% Magnesiummethanolat resultiert. Bevorzugt werden methanolische Lösungen mit einem Gehalt von 1 bis 15 Gew.-% Magnesiummethanolat, insbesondere 3 bis 10 Gew.-%ige Lösungen.

Festes Magnesiummethanolat kann man durch Eindampfen der nach dem erfindungsgemäßen Verfahren erhaltenen Lösung erhalten.

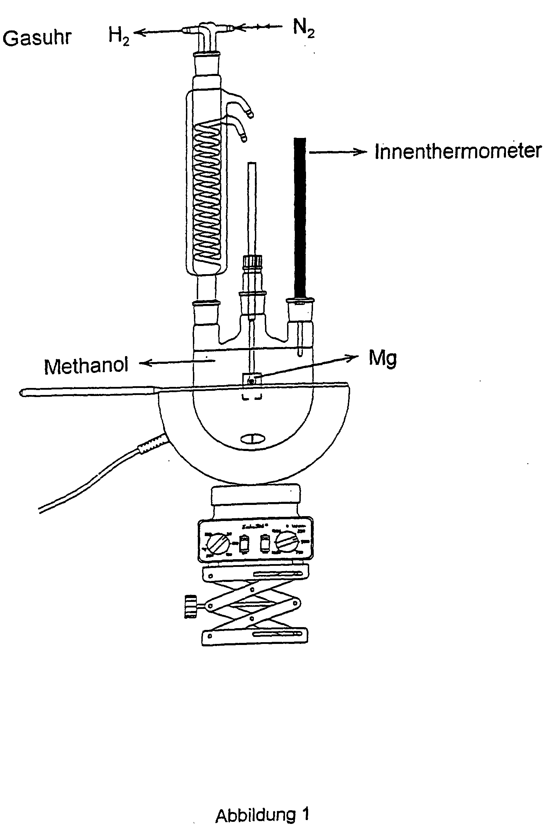

Beispielsweise kann man die Umsetzung der Edukte Methanol und Magnesium in einer Reaktionsapparatur, wie sie aus Abbildung 1 zu entnehmen ist, erfindungsgemäß durchführen.

Im Allgemeinen führt man das erfindungsgemäße Verfahren wie folgt aus:

Die Reaktion verläuft in der Regel gemäß Reaktionsgleichung 1:

$$Mg + 2CH_3OH \rightarrow Mg(OCH_3)_2 + H_2 \tag{1}$$

Als Nebenreaktionen können aber auch folgende Reaktionen auftreten:

$$Mg(OCH_3)_2 + 2H_2O \rightarrow Mg(OH)_2 + 2CH_3OH \tag{2}$$

$$Mg(OCH_3)_2 + CO_2 + H_2O \rightarrow MgCO_3 + 2CH_3OH \tag{3}$$

Daher sollte das erfindungsgemäße Verfahren unter Ausschluss von Wasser sowie Kohlendioxid durchgeführt werden. Maßnahmen zum Arbeiten unter Ausschluss von Wasser und Kohlendioxid sind im Allgemeinen bekannt.

Der für das vorliegende Verfahren verwendete Reaktor, z. B. ein Labor-Dreihalskolben, ist geeigneterweise ausgestattet mit einer Temperaturmessung, beispielsweise Innenthermometer, einer Gasvolumenmessung, beispielsweise Gasuhr, einem Rückflusskühler, einer Inertgasüberlagerung, beispielsweise mit trockenem Stickstoff, ggf. einer Magnesiumhaltevorrichtung, einer Heizung, beispielsweise Heizpilz, und einem Rührer, beispielsweise Flügelrührer oder Magnetrührer mit Magnetkern (vergleiche Abbildung 1), mit dessen Hilfe man während der Umsetzung stets für eine gute Durchmischung bzw. Umspülen des Magnesiums mit Methanol sorgt .

Üblicherweise wird zur Durchführung des Verfahrens die Apparatur zunächst mit einem trockenen Inertgas gespült, beispielsweise mit Stickstoff, und gleichzeitig ausgeheizt. Nun kann man das Magnesium - geeigneterweise unter Schutzgas - in den Reaktionsbereich der Anlage einbringen. Man kann das Magnesium im Reaktionsraum auch an einer Aufhängevorrichtung befestigen. Für die Inbetriebnahme des Rückflusskühlers ist als Kühlmedium beispielsweise Leitungswasser geeignet. Der Reaktor kann nun mit- vorzugsweise getrocknetem-Methanol so befüllt werden, dass das Magnesium allseitig von Methanol umspült ist. Geeigneterweise führt man die erfindungsgemäße Umsetzung unter Siedebedingungen durch. Das Methanol siedet unter Normalbedingungen bei rd. 64 °C. Die Umsetzung zwischen Methanol und stückigem Magnesium kann aber auch unter vermindertem oder erhöhtem Druck bei niedrigerer oder höherer Temperatur in einem dafür geeigneten Reaktor durchgeführt werden. Nach rund einer halben Stunde setzt im Allgemeinen die Reaktion des Methanols mit dem Magnesium ein, und an der Gasuhr kann deutlich die damit verbundene Wasserstoffentwicklung abgelesen werden. Die Umsetzung ist beendet, wenn alles Magnesium abreagiert ist und an der Gasuhr keine Wasserstoffentwicklung mehr angezeigt wird. Die Umsetzung läuft üblicherweise 1 bis 60 Stunden, vorzugsweise 12 ± 4 Stunden. Man kann das Verfahren aber auch vorher beenden. Erhalten wird in der Regel eine klare bis leicht trübe methanolische Lösung. Sie kann zusätzlich unter Schutzgas filtriert werden. Sofern ein festes Magnesiummethanolat als Produkt angestrebt ist, dampft man die nach dem erfindungsgemäßen Verfahren erhaltene Lösung ein. Hierzu kann der Druck von Umgebungsdruck, 1 bar abs., auf < 1 mbar abs. abgesenkt werden. Gleichzeitig kann die Temperatur von 64 °C auf beispielsweise 150 °C im Innern des Kolbens erhöht werden. In der Regel erzielt man beim erfindungsgemäßen Verfahren Produktausbeuten, bezogen auf das eingesetzte Magnesium, von > 95 %. Insbesondere werden Ausbeuten von 90 bis 100 % erzielt. Üblicherweise liegt die Reinheit der erfindungsgemäß hergestellten Produkte bei > 98 %, bezogen auf Magnesiummethanolat, vorzugsweise bei 98 bis 99,99 %.

Nach dem erfindungsgemäßen Verfahren erhaltenes Magnesiummethanolat kann sowohl als methanolische Lösung als auch in fester Form in hervorragender Weise als Einsatzstoff für chemische und pharmazeutische Prozesse, für die Herstellung von Katalysatoren, vorzugsweise für Polymerisationskatalysatoren, sowie als Neutralisationsmittel, insbesondere als wasserfreies Neutralisationsmittel, verwendet werden.

Die vorliegende Erfindung wird durch die nachfolgenden Beispiele näher erläutert:

Beispiele:

Beispiel 1

In eine wie in Abbildung 1 dargestellte und im Text beschriebene Apparatur mit einem Volumen von 2 l werden 29,7 g Mg in Blockform (Maße: 1,5 x 1,5 x 7,5 cm) und 1 317,8 g Methanol eingefüllt. Die Apparatur wird mit Stickstoff gespült, der Rückflusskühler mit Kühlwasser beaufschlagt und der Inhalt zum Sieden erhitzt (Siedetemperatur ca. 64 °C). Nach weiteren ca. 30 Minuten setzt die Reaktion ein, und an der Gasuhr ist die Wasserstoffentwicklung ablesbar. Die maximale Wasserstoffentwicklung beträgt 7 l/h. Nach ca. 12 Stunden ist die Reaktion abgeschlossen. Man erhält 1 291 g Magnesiummethylatlösung mit einem Mg-Gehalt von 2,2 Gew.-%. Die Summe aus $Mg(OH)_2$ und $MgCO_3$

beträgt 0,04 Gew.-%.
Ausbeute: 96 %.

Beispiel 2

Die nach Beispiel 1 erhaltene Lösung wird in einem Rotavapor überführt und dort unter verringertem Druck eingedampft. Die Trocknung ist beendet bei einer Temperatur von ca. 150°C und einem Druck von < 1 mbar. Man erhält ein weißes Pulver (97,6 g entsprechen einer Ausbeute von 97 %) mit einem Mg-Wert von 27,8 %.

Vergleichsbeispiel

In einem 2-l-Dreihalskolben mit Rückflusskühler, Innenthermometer und Rührer werden 22 g Mg-Späne und 1 000 g Methanol vorgelegt. Die Reaktion kommt von selbst in Gang, Dauer ca. 30 Minuten. Danach setzt eine sehr heftige Reaktion ein (starker Temperaturanstieg), die nur noch unter Einsatz von Trockeneiskühlung beherrscht werden kann. Nach weiteren ca. 25 Minuten flacht die Reaktion ab und das Magnesium reagiert vollends ab. Gesamtdauer der Reaktion: ca. 2,5 Stunden.

**Patentansprüche**

1. Verfahren zur Herstellung von Magnesiummethanolat durch Umsetzen von Magnesium mit Methanol,
   **dadurch gekennzeichnet,**
   **dass** man Magnesium in Stückform einsetzt.

2. Verfahren nach Anspruch 1,
   **dadurch gekennzeichnet,**
   **dass** eingesetzte Magnesiumstücke im Mittel eine geometrische Oberfläche von mindestens 10 cm$^2$ aufweisen.

3. Verfahren nach Anspruch 1 oder 2,
   **dadurch gekennzeichnet,**
   **dass** man ein einzelnes Magnesiumstück einsetzt, das eine geometrische Oberfläche von mindestens 10 cm$^2$ aufweist.

4. Verfahren nach mindestens einem der Ansprüche 1 bis 3,
   **dadurch gekennzeichnet,**
   **dass** das eingesetzte Magnesium Block-, Säulen- und/oder Plattenform besitzt.

5. Verfahren nach mindestens einem der Ansprüche 1 bis 4,
   **dadurch gekennzeichnet,**
   **dass** man die Umsetzung bei einer Temperatur im Bereich von 0 bis 200 °C durchführt.

6. Verfahren nach mindestens einem der Ansprüche 1 bis 5,
   **dadurch gekennzeichnet,**
   **dass** man die Umsetzung bei einem Druck im Bereich von 0,1 bis 100 bar abs. durchführt.

7. Verfahren nach mindestens einem der Ansprüche 1 bis 6,
   **dadurch gekennzeichnet,**
   **dass** man als Edukte flüssiges Methanol und festes Magnesium in einem solchen Mengenverhältnis einsetzt, dass daraus eine methanolische Lösung mit einem Gehalt von 0,1 bis 30 Gew.-% Magnesiummethanolat resultiert.

8. Verfahren nach mindestens einem der Ansprüche 1 bis 7,
   **dadurch gekennzeichnet,**
   **dass** man durch Eindampfen der erhaltenen Lösung festes Magnesiummethanolat erhält.

9. Verwendung des nach einem der Ansprüche 1 bis 8 erhaltenen Magnesiummethanolats als Einsatzstofl für chemische oder pharmazeutische Prozesse oder für die Herstellung von Katalysatoren oder als Neutralisationsmittel.

Abbildung 1

**Europäisches Patentamt**

# EUROPÄISCHER RECHERCHENBERICHT

Nummer der Anmeldung

EP 01 11 9327

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 3 094 546 A (SMITH TOWERS RUSSELL) 18. Juni 1963 (1963-06-18) * Spalte 7 - Spalte 8; Anspruch 1 * --- | 1-8 | C07C29/70 |
| X | DE 12 51 297 B (STAUFFER CHEMICAL COMPANY) 5. Oktober 1967 (1967-10-05) * Spalte 3 - Spalte 8 * --- | 1,4-8 | |
| X | FR 2 022 467 A (DYNAMIT NOBEL AG) 31. Juli 1970 (1970-07-31) * seite 1, absatz 1; seite 4; Beispiel 1 und Ansprüche * --- | 1,4-9 | |
| X | PATENT ABSTRACTS OF JAPAN vol. 002, no. 008 (C-001), 20. Januar 1978 (1978-01-20) & JP 52 111508 A (TOSHIBA CORP), 19. September 1977 (1977-09-19) * Zusammenfassung * ----- | 1 | |

**RECHERCHIERTE SACHGEBIETE (Int.Cl.7)**

C07C

Der vorliegende Recherchenbericht wurde für alle Patentansprüche erstellt

| Recherchenort | Abschlußdatum der Recherche | Prüfer |
|---|---|---|
| BERLIN | 21. September 2001 | Rufet, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 01 11 9327

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-09-2001

| Im Recherchenbericht angeführtes Patentdokument | | Datum der Veröffentlichung | Mitglied(er) der Patentfamilie | | Datum der Veröffentlichung |
|---|---|---|---|---|---|
| US 3094546 | A | 18-06-1963 | KEINE | | |
| DE 1251297 | B | | KEINE | | |
| FR 2022467 | A | 31-07-1970 | DE | 1806549 A1 | 14-05-1970 |
| | | | AT | 291933 B | 15-07-1971 |
| | | | BE | 741121 A | 01-04-1970 |
| | | | CH | 526584 A | 15-08-1972 |
| | | | ES | 373061 A1 | 16-11-1971 |
| | | | FR | 2022467 A5 | 31-07-1970 |
| | | | GB | 1228096 A | 15-04-1971 |
| | | | NL | 6916441 A | 06-05-1970 |
| | | | SE | 354842 B | 26-03-1973 |
| | | | US | 3657361 A | 18-04-1972 |
| JP 52111508 | A | 19-09-1977 | JP | 1032803 C | 20-02-1981 |
| | | | JP | 55022458 B | 17-06-1980 |

EPO FORM P0461

Für nähere Einzelheiten zu diesem Anhang : siehe Amtsblatt des Europäischen Patentamts, Nr.12/82